(19) Europäisches Patentamt
European Patent Office
Office européen des brevets

(11) Veröffentlichungsnummer **0 005 446**
**B1**

# (12) EUROPÄISCHE PATENTSCHRIFT

(45) Veröffentlichungstag der Patentschrift 15.09.82

(51) Int. Cl.³ **C 07 C 101/24**, C 07 C 143/14, C 11 D 1/88, A 61 K 7/08

(21) Anmeldenummer 79101077.0

(22) Anmeldetag 09.04.79

(54) **Amphotere oberflächenaktive Aminoetherverbindungen und Verfahren zu ihrer Herstellung.**

(30) Priorität 14.04.78 US 896294

(43) Veröffentlichungstag der Anmeldung
28.11.79 Patentblatt 79/24

(45) Bekanntmachung des Hinweises auf die Patenterteilung:
15.09.82 Patentblatt 82/37

(84) Benannte Vertragsstaaten
DE FR GB NL

(56) Entgegenhaltungen:
DE-A-2 137 061
DE-A-2 236 273
DE-B-1 013 289
FR-A-70 139
US-A-2 673 213
US-A-3 888 797

(73) Patentinhaber **Henkel Kommanditgesellschaft auf Aktien, Postfach 1100 Henkelstrasse 67, D-4000 Düsseldorf 1 (DE)**

(72) Erfinder: **Leenders, Peter, Allendale New Jersey 07401, Allendale New Jersey 07401 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

»Amphotere oberflächenaktive Aminoetherverbindungen und Verfahren zu ihrer Herstellung«

Die Erfindung betrifft Gemische aus amphoteren oberflächenaktiven Aminoetherverbindungen, die eine Diaminstruktur aufweisen, sowie ein Verfahren zur Herstellung dieser Verbindungsgemische.

In Waschmittelzusammensetzungen ist bereits eine große Anzahl von unterschiedlichen amphoteren oberflächenaktiven Verbindungen eingesetzt worden. Wegen ihrer geringen Augenreizwirkung sind diese Verbindungen von besonderem Interesse für die Verwendung in Haarshampooformulierungen. Man hat festgestellt, daß Produkte, die eine Diaminstruktur besitzen, die geringste Reizwirkung auf die Bindehaut ausüben.

Bei Produkten, die eine Diaminstruktur aufweisen, wird häufig angegeben, daß sie einen Imidazolinring besitzen, wie z. B. in der US-PS 2 849 315. In diesem US-Patent werden oberflächenaktive Imidazoline beschrieben, denen die Formel

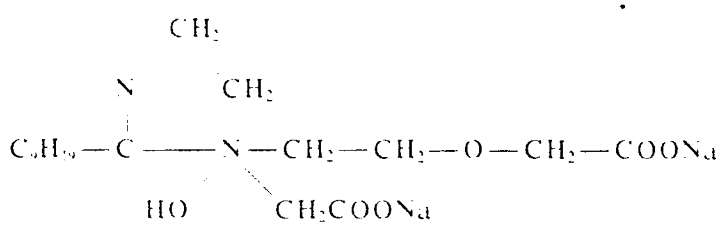

zukommt. Solche Imidazolinstrukturen sind jedoch nur unter Schwierigkeiten zu erhalten, da diese in Gegenwart von Wasser leicht hydrolysieren und eine lineare Struktur

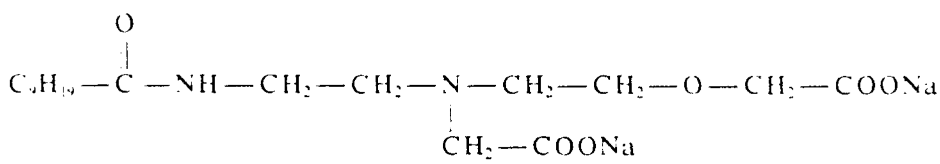

ergeben. Sowohl in alkalischer als auch in saurer Lösung zerfallen diese Produkte nach und nach, wobei sich ihre Lösungen im Laufe der Zeit trüben.

Es sind auch noch andere Typen von oberflächenaktiven Verbindungen mit Diaminstruktur beschrieben worden, beispielsweise in der US-PS 2 927 901, der US-PS 2 993 918, der US-PS 3 813 422 und der US-PS 3 888 797. Die dort beschriebenen Verbindungen unterliegen leicht der Zersetzung, sofern sie die Amidgruppierung $-CO-NH-$ enthalten, oder sie sind nicht ausreichend hydrophil, wenn nur eine Hydroxyl- oder Ethergruppierung vorhanden ist.

Der vorliegenden Erfindung lag die Aufgabe zugrunde, amphotere oberflächenaktive Verbindungen mit Diaminstruktur zu entwickeln, die über einen weiten pH-Bereich vom sauren bis in das alkalische Gebiet hinein über einen langen Zeitraum stabil sind und die mindestens drei Hydroxyl- und/oder Ethergruppen besitzen, damit das Molekül stärkere hydrophile Eigenschaften erhält.

Die der Erfindung zugrunde liegende Aufgabe wurde gelöst durch die Entwicklung eines neuen Gemisches aus amphoteren oberflächenaktiven Aminoetherverbindungen, das dadurch erhalten wird, daß man einen Glycidylether der Formel

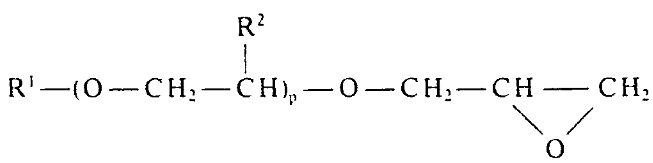

I

in der $R^1$ einen primären Alkylrest eines gesättigten Fettalkohols mit 8 bis 22 Kohlenstoffatomen, einen primären Alkenylrest eines ungesättigten Fettalkohols mit 12 bis 22 Kohlenstoffatomen oder einen in 2-Stellung verzweigten Alkylrest eines Guerbetalkohols mit 10 bis 36 Kohlenstoffatomen, $R^2$ ein Wasserstoffatom oder einen Methylrest und p eine ganze Zahl von 0 bis 3 bedeuten, mit einem im Überschuß vorhandenen N-Hydroxy-$C_{2-4}$-alkyl-$C_{2-6}$-alkylendiamin umsetzt, das überschüssige Diamin entfernt und den erhaltenen substituierten Aminoether mit einem N-Alkylierungsmittel aus der Gruppe Halogen-$C_{2-4}$-alkancarbonsäuren, Halogen-$C_{2-4}$-alkansulfonsäuren und Halogen-hydroxy-$C_{3-4}$-alkansulfonsäuren unter alkalischen Bedingungen umsetzt, wobei das Molverhältnis von Aminoether zu N-Alkylierungsmittel 1 : 1,4 bis 1 : 4 beträgt.

Die Verbindungen des auf diese Weise erhaltenen Gemisches enthalten ein Minimum von 3 Hydroxyl- und/oder Ethergruppen und keine Amidgruppierung; aus diesem Grund sind sie leicht löslich in Wasser und hydrolysestabil.

Gegenstand der Erfindung ist weiterhin das Verfahren zur Herstellung der neuen Gemische aus amphoteren oberflächenaktiven Aminoetherverbindungen auf dem oben beschriebenen Weg.

In den als Ausgangsmaterial verwendeten Glycidylethern der Formel I hat p vorzugsweise den Wert 0; $R_1$ ist vorzugsweise ein Alkylrest mit 8 bis 18 Kohlenstoffatomen. Diese Glycidylether können leicht dadurch erhalten werden, daß man einen entsprechenden Alkohol mit Epichlorhydrin umsetzt und den erhaltenen Ether unter alkalischen Bedingungen dehydrohalogeniert. Diese Glycidylether stellen handelsübliche Produkte dar. Als Beispiele für geeignete Verbindungen der Formel I seien Lauryl-, Myristyl-, Palmityl-, Stearyl- und Oleylglycidylether, ein überwiegend $C_{10}$- und $C_{12}$-Alkylreste enthaltendes Glycidylethergemisch, die Glycidylether von technischen Fettalkoholen wie Kokosfettalkohol, hydriertem Kokosfettalkohol, Talgfettalkohol und hydriertem Talgfettalkohol sowie der Glycidylether eines $C_{24}$-Guerbetalkohols genannt.

Die weiterhin als Ausgangsmaterial benutzten N-Hydroxy-$C_{2-4}$-alkyl-$C_{2-6}$-alkylendiamine sind ebenfalls im Handel erhältlich. Als Beispiele für Diamine, die in der Umsetzung verwendet werden können, seien N-Hydroxyethylethylendiamin, N-Hydroxypropylethylendiamin, N-Hydroxybutylethylendiamin, N-Hydroxyethylpropylendiamin, N-Hydroxypropylpropylendiamin und N-Hydroxyethylhexamethylendiamin genannt. N-Hydroxyethylethylendiamin wird bevorzugt eingesetzt.

Die als N-Alkylierungsmittel eingesetzten Halogen-$C_{2-4}$-alkancarbonsäuren, Halogen-$C_{2-4}$-alkansulfonsäuren und Halogenhydroxy-$C_{3-4}$-alkansulfonsäuren enthalten als Halogen vorzugsweise Chlor. Diese Säuren werden bevorzugt in Form ihrer Alkalimetallsalze eingesetzt, da die Reaktion unter alkalischen Bedingungen durchgeführt wird. Als N-Alkylierungsmittel werden vorzugsweise Natriumchloracetat, Natriumchlorpropionat, Natriumchlorethansulfonat und Natrium-3-chlor-2-hydroxypropansulfonat eingesetzt.

Die erste Reaktion, zwischen dem Alkylglycidylether und dem Diamin, wird unter den Bedingungen eines substanziellen Diamin-Überschusses durchgeführt, um die Bildung von di- und trisubstituierten Diaminen zurückzudrängen. Die Anwesenheit von größeren Mengen dieser Substanzen bei der N-Alkylierungsreaktion verursacht eine Trübung in den Reaktionsprodukten. Pro Mol Glycidylether werden 1,2 bis 4 Mol, vorzugsweise 1,5 bis 3 Mol und insbesondere 2 Mol Diamin eingesetzt.

Die bei erhöhter Temperatur durchgeführte Reaktion ist exotherm. Normalerweise wird die Reaktion in Abwesenheit eines Lösungsmittels durchgeführt. Wenn größere Substanzmengen umgesetzt werden müssen, kann es zweckmäßig sein, mit einem niedrigsiedenden Lösungsmittel bei Rückflußtemperatur zu arbeiten, um die entstehende Reaktionswärme unter Kontrolle zu halten. Die Reaktionstemperatur sollte 175°C, vorzugsweise 150°C nicht überschreiten. Demzufolge wird die Reaktion in einem geschlossenen Gefäß durchgeführt, das zum Einleiten der Reaktion geheizt und zum Abführen der Reaktionswärme gekühlt werden kann.

Nach dem Ende der Reaktion wird das überschüssige Diamin durch Vakuumdestillation entfernt. Das Reaktionsprodukt ist ein hellfarbiges, bei Raumtemperatur pastenförmiges oder wachsartiges Material, das in heißem Wasser leicht getrübte Lösungen ergibt.

Nach dem Reaktionsschema

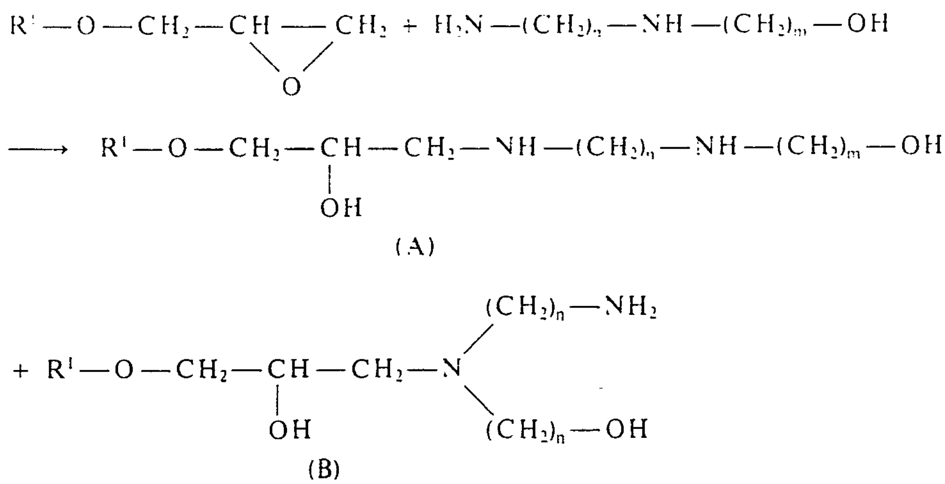

(A)

(B)

entstehen hauptsächlich 2 monosubstituierte Aminoether. Wenn das Diamin in ausreichendem Überschuß eingesetzt wird, dann entspricht der größere Teil des Reaktionsproduktes der Formel A. Es wird angenommen, daß die Menge des Produktes der Formel B im Reaktionsprodukt zwischen 1 und 10 Gewichtsprozent ausmacht.

Nach dem Abdestillieren des überschüssigen Diamins wird das erhaltene Gemisch aus den Produkten der Formeln A und B durch Erhitzen auf Temperaturen zwischen 50 und 98°C verflüssigt. Danach wird ein Überschuß des N-Alkylierungsmittels, in Wasser gelöst, zugegeben, wobei der pH-Wert durch langsame Zugabe von Alkalimetallhydroxidlösung auf der alkalischen Seite gehalten und gleichzeitig eine Temperatur von 70 bis 95°C aufrechterhalten wird. Der pH-Wert des

Reaktionsgemisches wird während der Reaktion vorzugsweise im Bereich von 7 bis 9, insbesondere im Bereich von 7 bis 8 gehalten.

Die eingesetzte Menge des N-Alkylierungsmittels beträgt 1,4 bis 4 Mol pro Mol des substituierten Aminoetherproduktes; vorzugsweise werden 2 bis 3,5 Mol, insbesondere 2,5 Mol eingesetzt. Um eine unnötige Verdünnung des Reaktionsgemisches nach dem Ende der Reaktion zu vermeiden, wird zum Lösen des Alkylierungsmittels nur eine begrenzte Menge Wasser verwendet. Vorzugsweise wird nur soviel Wasser angewendet, daß das Reaktionsprodukt zwischen 45 und 60 Gewichtsprozent Wasser enthält.

Das auf diese Weise erhaltene Reaktionsprodukt ist eine klare, leichtgelb gefärbte Flüssigkeit, die als solche für flüssige Shampoozusammensetzungen verwendet werden kann. Wenn ein chlorhaltiges Alkylierungsmittel verwendet wurde, enthält das Produkt in Abhängigkeit von dem Überschuß des Alkylierungsmittels etwa 10 Gewichtsprozent Natriumchlorid. Das erhaltene Produkt hat einen pH-Wert von 8 bis 9; es kann mit einer Säure behandelt werden, wobei es sich unter diesen Bedingungen wie eine typisch amphotere Verbindung verhält; bei niederen pH-Werten kann es kationische Eigenschaften haben.

Die N-Alkylierungsreaktion läuft nach dem folgenden Schema ab:

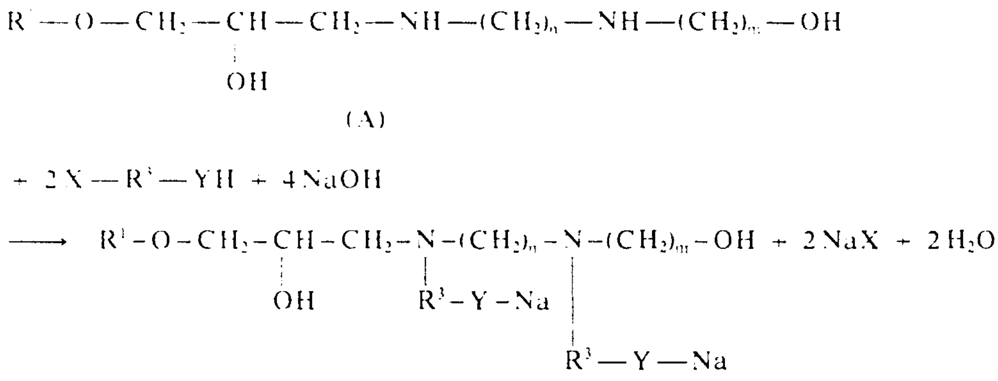

(A)

(C)

In geringem Ausmaß bleibt die Reaktion bei der Addition von einem Mol Alkylierungsmittel an eines der beiden Stickstoffatome stehen. In geringem Umfang tritt auch eine Dreifachaddition ein, bei der die primäre Hydroxylgruppe verethert wird. Weiterhin geht die in geringer Menge vorhandene Verbindung B analoge Reaktionen ein. Demzufolge schließt das Endprodukt die Möglichkeit von etwa 8 verschiedenen Reaktionsprodukten und die der unveränderten Verbindungen der Formeln A und B ein. In der Hauptsache besteht das Reaktionsprodukt jedoch aus dem Diadditionsprodukt der Formel C.

Wenn man einen $C_{8-18}$-Alkylglycidylether mit einem substanziellen Überschuß an N-Hydroxyethylethylendiamin umsetzt, anschließend das überschüssige Diamin entfernt und den entstandenen substituierten Aminoether mit einem Überschuß an Chloressigsäure umsetzt, so erhält man ein Gemisch von amphoteren oberflächenaktiven Aminoetherverbindungen, das Verbindungen der folgenden Formeln enthält

1) 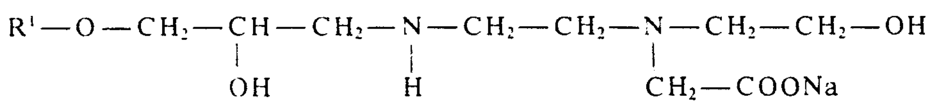

2) 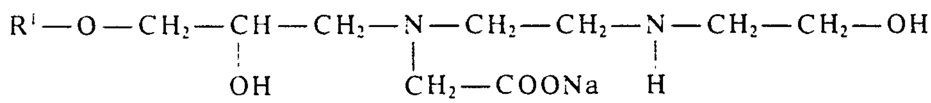

3) 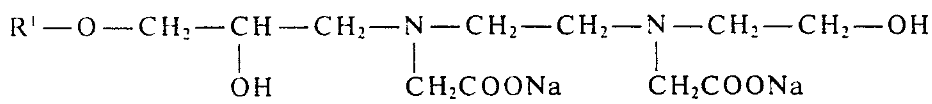

4) 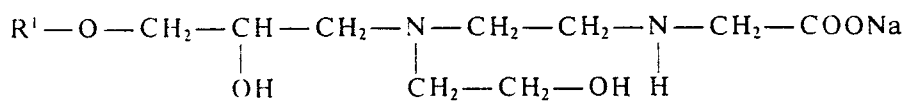

5) $$\begin{array}{l} R^1-O-CH_2-\underset{\displaystyle OH}{\underset{|}{CH}}-CH_2-\underset{\displaystyle CH_2-CH_2-OH}{\underset{|}{N}}-CH_2-CH_2-\underset{\displaystyle CH_2-COONa}{\underset{|}{N}}-CH_2-COONa \end{array}$$

($R^1 = C_{8-18}$-Alkyl),

wobei das Produkt der Formel 3 mehr als 50 Gewichtsprozent dieses Gemisches aus amphoteren oberflächenaktiven Aminoethern ausmacht.

Die nachfolgenden Beispiele sollen den Gegenstand der Erfindung erläutern, ihn jedoch nicht hierauf beschränken

## Beispiele

### Beispiel 1

#### Reaktionsstufe A

572 g (etwa 2 Mol) eines $C_{12-14}$-Alkylglycidylethers mit einem mittleren Molekulargewicht von 286 und 416 g (etwa 4 Mol) N-Hydroxyethylethylendiamin wurden in einen 2-l-Dreihalskolben mit Rührer und Rückflußkühler gebracht. In den Kolben wurde Stickstoff eingeleitet. Das Reaktionsgemisch wurde unter Rühren im Verlauf von 30 Minuten auf 70°C erhitzt. Bei dieser Temperatur setzte die exotherme Reaktion ein. Die Reaktionstemperatur stieg nun rasch auf 140°C an. Durch Kühlen mit einem Wasserbad wurde die Reaktionstemperatur 10 Minuten lang bei 140°C gehalten. Danach wurde das Reaktionsgemisch auf 150°C erhitzt und eine Stunde lang bei dieser Temperatur gehalten.

Das überschüssige N-Hydroxyethylethylendiamin wurde durch Vakuumdestillation bei 150 bis 170°C und einem von 20 auf 3 mbar abnehmenden Druck entfernt. Als Destillat wurden 239 g einer blaßgrünen Flüssigkeit mit 86,5 Gew.-% Hydroxyethylethylendiamin und 2,1 Gew.-% Wasser erhalten. Als Rückstand verblieben 727 g Reaktionsprodukt. Nach dem Abkühlen auf 60°C lag das Produkt als hellgelbe Paste vor. Nach der erhaltenen Destillatmenge bestand das Reaktionsprodukt fast ausschließlich aus dem Addukt von 1 Mol Glycidylether an 1 Mol N-Hydroxyethylethylendiamin. Das Reaktionsprodukt löste sich bei 90°C in Wasser und ergab eine leicht getrübte Lösung. Der Gehalt an Epoxidsauerstoff lag unter 1 Gew.-%.

#### Reaktionsstufe B

390 g (etwa 1 Mol) des Produktes aus der Reaktionsstufe A wurde in einen 2-l-Dreihalskoben mit Rührer, Tropftrichter und Rückflußkühler gebracht. Das Reaktionsprodukt wurde auf 70°C erwärmt bis es flüssig wurde. 298 g (etwa 2,5 Mol) Natriumchloracetat wurden in 688 g Wasser gelöst. Diese Wassermenge reichte aus, um eine 50gewichtsprozentige Lösung der Reaktionspartner zu ergeben. Die Lösung wurde dem geschmolzenen Reaktionsprodukt zugegeben. Anschließend wurde auf 80°C erhitzt. In das Reaktionsgemisch wurde eine pH-Elektrode eingetaucht, wobei ein pH-Wert von 8 festgestellt wurde. Durch tropfenweises Zugeben einer 50gewichtsprozentigen NaOH-Lösung wurde der pH-Wert bei etwa 7 bis 8 gehalten. Im Verlauf von 100 Minuten wurden 152 g (etwa 2 Mol) NaOH-Lösung zugegeben, während gleichzeitig die Temperatur langsam auf 95°C gesteigert wurde.

Die Temperatur wurde anschließend unter Rühren weitere 100 Minuten lang bei 95°C gehalten. Der pH-Wert veränderte sich hierbei nicht; was anzeigte, daß die Reaktion bereits zu Ende war. Es wurden 1481 g einer Lösung eines Gemisches aus amphoteren oberflächenaktiven Verbindungen als klare, hellbernsteinfarbige Flüssigkeit mit 9,6 Gew.-% NaCl (etwa 100% der Theorie) und 52,6 Gew.-% Wasser erhalten. Der pH-Wert einer 10gewichtsprozentigen Lösung bei Raumtemperatur war 8,6. Nach einer Lagerzeit von 2 Jahren konnte keine Veränderung der Eigenschaften festgestellt werden.

#### Bewertung

Das Verbindungsgemisch ergibt klare, stabile Lösungen in Wasser, 20gewichtsprozentiger Schwefelsäure, 20gewichtsprozentiger Natronlauge und in typischen Shampoozusammensetzungen in Kombiantion mit Fettalkoholethersulfaten.

Das Produkt wurde auf seine physikalischen und chemischen Eigenschaften, sein Verhalten in Standardrezepturen und in bezug auf Augenreizung hin untersucht. Diese Ergebnisse wurden mit den Ergebnissen verglichen, die mit einer bekannten amphoteren oberflächenaktiven Substanz (Vergleichssubstanz) erhalten wurden, von der angenommen wird, daß sie der Formel

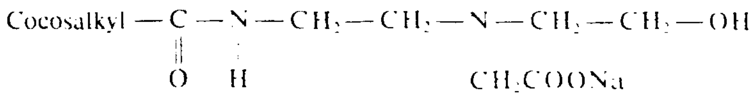

entspricht, und die in einer wäßrigen Lösung mit einem Feststoffgehalt von 40 Gew.-% (9 Gew.-% NaCl) vorliegt.

A) Physikalische und chemische Eigenschaften

1. Analytische Werte

| | |
|---|---|
| Wasser | 52,6 Gew.-% |
| Chlorid | 9,6 Gew.-% |
| pH (10gewichtsprozentige Lösung) | 8,6 |
| Viskosität (Brookfield; Spindel Nr. 3, 10 UpM; 25° C) | 3800 mPa s |

2. Stabilität in Säuren, Basen und Salzlösungen

| | |
|---|---|
| Säurestabilität ($H_2SO_4$; 20gewichtsprozentige Lösung) | keine Trennung, Trübung oder Sediment bei 10 Gew.-% |
| Basenstabilität (NaOH; 20gewichtsprozentige Lösung) | keine Trennung, Trübung oder Sediment bei 10 Gew.-% |
| Salzstabilität (NaCl; 20gewichtsprozentige Lösung) | keine Trennung, Trübung oder Sediment bei 10 Gew.-% |

B) Vergleichende Untersuchungen

1. Schaumverhalten
(modifizierter Ross-Miles-Test)

a) Testbedingungen:

100 ml Lösung mit 0,1 Gew.-% Aktivsubstanz, 25°C, Leitungswasser

| Produkt | Schaumhöhe (ml) |
|---|---|
| Beispiel 1 | 250/235 |
| Vergleichssubstanz | 255/240 |

b) Testbedingungen:

100 ml Lösung mit 0,1 Gew.-% Aktivsubstanz, 25°C, Leitungswasser

| Produkt | Schaumhöhe (ml) |
|---|---|
| Beispiel 1 | 350/325 |
| Vergleichssubstanz | 350/325 |

C) Shampoo-Zusammensetzungen

Das Produkt aus Beispiel 1 wurde in bezug auf Viskosität, Schaumverhalten und Trübungspunkt bewertet.

Shampoo Nr. 1

| Bestandteil | A | B |
|---|---|---|
| Wasser | 57,5 | 57,5 |
| Propylenglykol | 1,0 | 1,0 |
| Cocosfettsäurediethanolamid (70 Gew.-%) + Glycerin (30 Gew.-%) | 0,5 | 0,5 |
| Na-Laurylsulfat (30 Gew.-% Aktivsubstanz) | 16,0 | 16,0 |
| Substanz aus Beispiel 1 | 25,0 | – |
| Vergleichssubstanz | – | 25,0 |
| pH (eingestellt) | 7,0 | 7,0 |
| Viskosität (mPa · s) | 20 000 | 880 |
| Schaumverhalten | 185/175 | 180/165 |
| Trübungspunkt | unter 0°C | unter 0°C |

Shampoo Nr. 2

| Bestandteil | A | B |
|---|---|---|
| Wasser | 62,5 | 62,5 |
| Natriumchlorid | 1,0 | 1,0 |
| Na-$C_{12-14}$-Fettalkoholdiethylenglykolethersulfat | 10,0 | 10,0 |
| Sulfat eines Additionsproduktes aus $C_{12-15}$-Synthesealkohol + 12 EO; Na-Salz (30 Gew.-% Aktivsubstanz) | 12,0 | 12,0 |
| Substanz aus Beispiel 1 | 12,0 | – |
| Vergleichssubstanz | – | 12,0 |
| pH (eingestellt) | 7,0 | 7,0 |
| Viskosität (mPa · s) | 350 | 200 |
| Schaumverhalten | 170/155 | 165/160 |
| Trübungspunkt | unter 0°C | unter 0°C |

Aus den oben angeführten Daten geht hervor, daß sich die beiden Substanzen in der Shampoozusammensetzung Nr. 2 annähernd gleich verhalten. In der Shampoozusammensetzung Nr. 1 tritt ein merklicher Viskositätsunterschied auf; dies wird jedoch nicht als gravierend angesehen.

D) Untersuchung der Reizwirkung

| Produkt | Beobachtung |
|---|---|
| Substanz aus Beispiel 1 (volle Stärke) | Bindehautreizung, die am siebten Tag verschwand |
| Vergleichssubstanz (volle Stärke) | Bindehautreizung, die am siebten Tag verschwand |
| Substanz aus Beispiel 1 (25% Aktivsubstanz) | Bindehautreizung, die am dritten Tag verschwand |
| Vergleichssubstanz (25% Aktivsubstanz) | Bindehautreizung, die am siebten Tag verschwand |

Bei der Prüfung der Augenreizwirkung war die Substanz aus Beispiel 1 zufriedenstellend; sie verhielt sich besser als die in Shampoos verwendete amphotere Standardverbindung.

E) Hydrolyseuntersuchungen

Proben der Vergleichssubstanz und der Substanz aus Beispiel 1 wurden in 20gewichtsprozentiger Natriumhydroxidlösung und 20gewichtsprozentiger Schwefelsäurelösung sieben Tage lang zum Rückflußkochen erhitzt. Am Ende des Experimentes waren die Lösungen der Vergleichssubstanz sichtbar verändert, während die Lösungen der Substanz aus Beispiel 1 unverändert erschienen. Auf der sauren Hydrolyselösung der Vergleichssubstanz schwamm ein Festprodukt, die alkalische Hydrolyselösung dieses Produktes enthielt ein gelatineartiges Festprodukt.

Die alkalische Hydrolyselösung wurde bis zu einem pH von 3 angesäuert und auf eine Konzentration von etwa 1 Gew.-% (Anfangskonzentration = 5 Gew.-%) verdünnt und die Oberflächenspannung mit der Stalagmometermethode bestimmt. Dasselbe Verfahren wurde für die alkalische Hydrolyselösung der Substanz aus Beispiel 1 angewandt. Diese Lösung enthielt jedoch kein Festprodukt.

Die sauren Hydrolyselösungen wurden auf eine Konzentration von 1 Gew.-% Aktivsubstanz verdünnt; anschließend wurde ihre Oberflächenspannung gemessen. Auch hier war in der Lösung der Vergleichssubstanz ein Festkörper vorhanden, während die Lösung der Substanz aus Beispiel 1 frei von Festprodukten war.

Die zu Beginn und am Ende des Experimentes gemessenen Oberflächenspannungen sind in der nachstehenden Tabelle wiedergegeben.

Tabelle

Oberflächenspannung vor und nach der Hydrolyse

| Substanz | Lösung | Oberflächenspannung (mN/m) vorher | nachher |
|---|---|---|---|
| Beispiel 1 | 20% NaOH | 66,4 | 65,5 |
| Beispiel 1 | 20% $H_2SO_4$ | 34,2 | 32,1 |
| Vergleich | 20% NaOH | 55,9 | 64,6 |
| Vergleich | 20% $H_2SO_4$ | 19,9 | 54,4 |

Die Oberflächenspannungen lassen erkennen, daß bei der Vergleichssubstanz in beiden Lösungen eine beträchtliche Zersetzung eingetreten ist. Das offensichtliche Vorhandensein von Seife bzw. Fettsäure bestätigen diesen Schluß. Beide Erscheinungen traten im Fall des Produktes aus Beispiel 1 nicht auf.

Beispiel 2

In einen Glaskolben mit Rührer, Rückflußkühler und Tropftrichter wurde etwa 1 Mol des Reaktionsproduktes aus Schritt A des Beispiels 1 gebracht. Das Produkt wurde auf 70°C erwärmt, um es zu verflüssigen. Anschließend wurden 2,5 Mol Natrium-2-hydroxy-3-chlorpropansulfonat als etwa 50gewichtsprozentige wäßrige Lösung bei 70°C zugegeben. Das Natrium-2-hydroxy-3-chlorpropansulfonat war durch Reaktion von Epichlorhydrin mit Natriumbisulfit erhalten worden. Die Temperatur der Lösung wurde auf 70—90°C gehalten, während 2 Mol Natriumhydroxid als 50gewichtsprozentige Lösung zugegeben wurden. Nach dem Abkühlen wurde eine Lösung eines Gemisches aus amphoteren oberflächenaktiven Substanzen mit einem Gehalt von etwa 40 Gew.-% Aktivsubstanz erhalten. Die aktiven Verbindungen hatten wahrscheinlich die Formeln

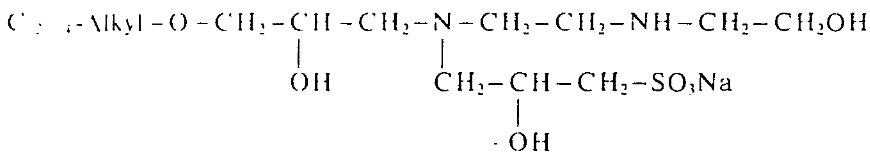

und

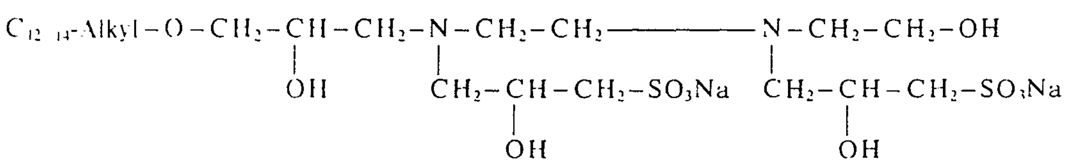

wobei die letztere wahrscheinlich überwiegt. Das Produkt hat Stabilitätswerte und oberflächenaktive Eigenschaften, die denen des Produktes aus Beispiel 1 vergleichbar sind.

**Patentansprüche**

1. Ein Gemisch aus amphoteren, oberflächenaktiven Aminoetherverbindungen, dadurch erhältlich, daß man einen Glycidylether der Formel

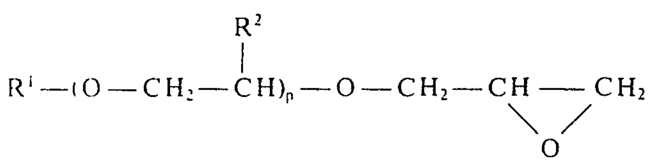 I

in der $R^1$ einen primären Alkylrest eines gesättigten Fettalkohols mit 8 bis 22 Kohlenstoffatomen, einen primären Alkenylrest eines ungesättigten Fettalkohols mit 12 bis 22 Kohlenstoffatomen oder einen in 2-Stellung verzweigten Alkylrest eines Guerbetalkohols mit 10 bis 36 Kohlenstoffatomen, $R^2$ ein Wasserstoffatom oder einen Methylrest und p eine ganze Zahl von 0 bis 3 bedeuten, mit einem im Überschuß vorhandenen N-Hydroxy-$C_{2-4}$-alkyl-$C_{2-6}$-alkylendiamin umsetzt, das überschüssige Diamin entfernt und den erhaltenen substituierten Aminoether mit einem N-Alkylierungsmittel aus der Gruppe Halogen-$C_{2-4}$-alkancarbonsäuren, Halogen-$C_{2-4}$-alkansulfonsäuren und Halogen-hydroxy-$C_{3-4}$-alkansulfonsäuren unter alkalischen Bedingungen umsetzt, wobei das Molverhältnis von Aminoether zu N-Alkylierungsmittel 1 : 1,4 bis 1 : 4 beträgt.

2. Verfahren zur Herstellung des Gemisches aus amphoteren oberflächenaktiven Aminoetherverbindungen nach Anspruch 1, dadurch gekennzeichnet, daß man einen Glycidylether der Formel

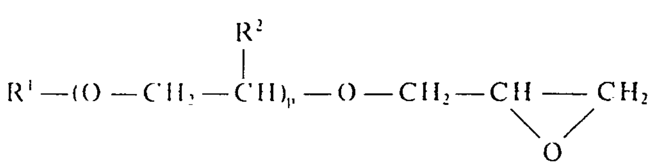 I

in der $R^1$, $R^2$ und p die im Anspruch 1 angegebene Bedeutung haben, mit einem im Überschuß vorhandenen N-Hydroxy-$C_{2-4}$-alkyl-$C_{2-6}$-alkylendiamin umsetzt, das überschüssige Diamin entfernt und den erhaltenen substituierten Aminoether mit einem N-Alkylierungsmittel aus der Gruppe Halogen-$C_{2-4}$-alkancarbonsäuren, Halogen-$C_{2-4}$-alkansulfonsäuren und Halogenhydroxy-$C_{3-4}$-alkansulfonsäuren unter alkalischen Bedingungen umsetzt, wobei das Molverhältnis von Aminoether zu N-Alkylierungsmittel 1 : 1,4 bis 1 : 4 beträgt.

## Claims

1. A mixture of amphoteric surface-active aminoether compounds obtainable by reacting a glycidyl ether corresponding to the following formula

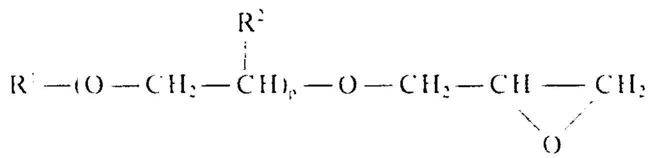

I

in which
$R^1$ represents a primary alkyl radical of a saturated fatty alcohol containing from 8 to 22 carbon atoms, a primary alkenyl radical of an unsaturated fatty alcohol containing from 12 to 22 carbon atoms or an alkyl radical branched in the 2-position of a guerbetalcohol containing from 10 to 36 carbon atoms, $R^2$ represents a hydrogen atom or a methyl radical and p is an integer of from 0 to 3, with an N-Hydroxy-$C_{2-4}$-alkyl-$C_{2-6}$-alkylene diamine present in excess, removing the excess diamine and reacting the substituted aminoether obtained with an N-alkylating agent from th group comprising halogen-$C_{2-4}$-alkane carboxylic acids, halogen-$C_{2-4}$-alkane sulfonic acids and halogenhydroxy-$C_{3-4}$-alkane sulfonic acids under alkaline conditions, the molar ratio of aminoether to N-alkylating agent amounting to between 1 : 1.4 and 1 : 4.

2. A process for producing the mixture of amphoteric surface-active aminoether compounds claimed in Claim 1, characterised in that a glycidyl ether corresponding to the following formula

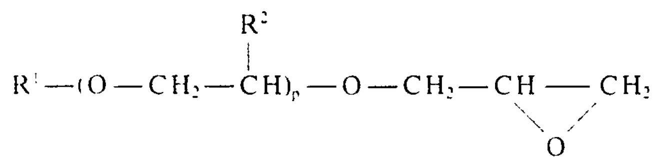

I

in which
$R^1$, $R^2$ and p are as defined in Claim 1, is reacted with an N-hydroxy-$C_{2-4}$-alkyl-$C_{2-6}$-alkylene diamine present in excess, the excess diamine is removed and the substituted aminoether obtained is reacted under alkaline conditions with an N-alkylating agent from the group comprising halogen-$C_{2-4}$-alkane carboxylic acids, halogen-$C_{2-4}$-alkane sulfonic acids and halogenhydroxy-$C_{3-4}$-alkane sulfonic acids, the molar ratio of aminoether to N-alkylating agent amounting to between 1 : 1.4 and 1 : 4.

## Revendications

1. Un mélange d'aminoéthers tensio-actifs amphotères, qui peut être obtenu par réaction d'un éther glycidylique de formule

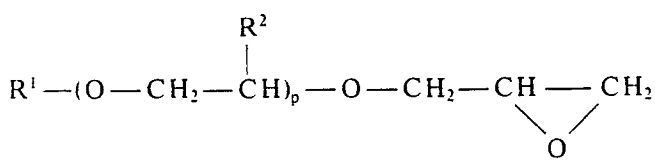

I

dans laquelle $R^1$ représente un groupe alkyle primaire d'un alcool gras saturé en C—C22, un groupe alkylène primaire d'un alcool gras insaturé C12—C22 ou un groupe alkyle ramifié en position 2 d'un alcool de Guerbet en C10—C36, $R^2$ représente un atome d'hydrogène ou un groupe méthyle et p est un nombre entier allant de 0 à 3, avec un excès d'une N-hydroxy-(alkyle en C2—C4)-(alkylène en C2—C6)-diamine, élimination de l'excès de diamine et réaction des aminoéthers substitués ainsi obtenus avec un agent N-alkylant pris dans le groupe des acides halogéno-(alcane en C2—C4)-carboxyliques, les acides halogéno-(alcane en C2—C4)-sulfoniques et les acides halogénohydroxy-(alcane en C3—C4)-sulfoniques en milieu alcalin, avec un rapport molaire aminoéther/agent N-alkylant de 1 : 1,4 à 1 : 4.

2. Procedé de préparation du mélange d'aminoéthers tensio-actifs amphotères selon la revendication 1, caractérisé en ce que l'on fait réagir un éther glycidylique de formule

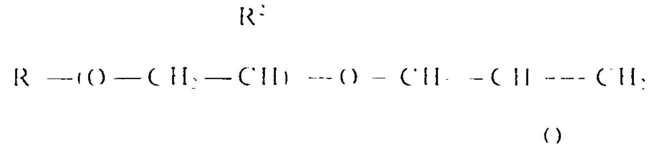

I

dans laquelle R', $R^2$ et p ont les significations indiquees dans la revendication 1, avec un excès d'une N-hydroxy-(alkyle en C2—C4)-(alkylene en C2—C6)-diamine, on élimine la diamine en excès et on fait réagir les aminoéthers substitués ainsi obtenus avec un agent N-alkylant pris dans le groupe des acides halogéno-(alcane en C2—C4)-carboxyliques, les acides halogéno-(alcane en C2—C4)-sulfoniques et les acides halogénohydroxy-(alcane en C3—C4)-sulfoniques en milieu alcalin, avec un rapport molaire aminoéther/agent N-alkylant de 1 : 1,4 à 1 : 4.